(19) Europäisches Patentamt
European Patent Office
Office européen des brevets

(11) Numéro de publication: **0 058 111**
**B1**

# (12) FASCICULE DE BREVET EUROPEEN

(45) Date de publication du fascicule du brevet: **23.10.85**

(51) Int. Cl.⁴: **C 07 D 209/20**, A 61 K 31/40

(21) Numéro de dépôt: **82400165.5**

(22) Date de dépôt: **29.01.82**

(54) **5-acétyloxy-L-tryptophane et ses sels d'addition, procédé de préparation et utilisation en thérapeutique.**

(30) Priorité: **04.02.81 FR 8102172**

(43) Date de publication de la demande:
**18.08.82 Bulletin 82/33**

(45) Mention de la délivrance du brevet:
**23.10.85 Bulletin 85/43**

(84) Etats contractants désignés:
***AT BE CH DE FR GB IT LI LU NL SE***

(56) Documents cités:
**FR - A - 1 457 260**
**FR - A - 2 395 995**
**GB - A - 2 001 309**

**CHEMICAL ABSTRACTS, vol. 54, no. 16, 25 août 1960, colonne 17725h Columbus, Ohio, USA S. COURVOISIER et al.: "Protective action of parenterally adminstered 5-hydroxy-tryptamine (5-HT) toward bronchospasms induced in the guinea pig by (5-HT) aerosols; specificity of this action"**

**Le dossier contient des informations techniques présentées postérieurement au dépôt de la demande et ne figurant pas dans le présent fascicule.**

(73) Titulaire: **Laboratoires Merck-Clévenot, 5-9 Rue Anquetil, F-94130 Nogent-sur-Marne (FR)**

(72) Inventeur: **Viret, Jacques, 89 B, route de Florissant, CH-1206 Genève (CH)**

(74) Mandataire: **Corre, Jacques Denis Paul, Cabinet Reglmbeau 26, Avenue Kléber, F-75116 Paris (FR)**

**EP 0 058 111 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention concerne en tant que produits industriels nouveaux le 5-acétyloxy-L-tryptophane et ses sels d'addition. Elle concerne également le procédé de préparation de ces produits et leur utilisation en thérapeutique et notamment en neuropsychiatrie.

La préparation du 5-acétyloxy-L-tryptophane et de ses sels d'addition n'a jamais été décrite jusqu'à ce jour.

On sait que W. SAKAMI et G. TOENNIES, J. Biol. Chem., 144, 203 (1942) ont proposé une méthode d'acétylation des fonctions alcools d'hydroxyaminoacides, que cette méthode a été appliquée avec succès pour l'O-acétylation de la L-hydroxyproline, de la DL-sérine, de la DL-thréonine et de la L-tyrosine, mais qu'elle n'a pas pu être appliquée à l'O-acétylation d'un hydroxyaminoacide indolique tel que l'hydroxytryptophane. Ceci s'explique par le fait que l'un des co-auteurs de l'article susvisé [voir à cet effet G. TOENNIES et J. J. KOLB, J. Biol. Chem., 144, 219 (1942)] dit avoir observé que l'acétylation a lieu sur l'atome d'azote indolique.

La O-acétylation selon l'invention se distingue également de la N-acétylation du groupe $NH_2$ du 5-hydroxy-L-tryptophane qui est notamment illustrée par le brevet français n° 71-37 901 (publication n° 2 113 077).

Selon l'invention on préconise une méthode de O-acétylation permettant d'obtenir le 5-acétyloxy-L-tryptophane et ses sels d'addition. De plus le 5-acétyloxy-L-tryptophane et ses sels d'addition s'étant révélés utiles en thérapeutique et notamment en neuropsychiatrie, on préconise selon l'invention une composition thérapeutique qui renferme, en association avec un excipient physiologiquement acceptable, au moins un composé choisi parmi l'ensemble constitué par le 5-acétyloxy-L-tryptophane et ses sels d'addition.

L'invention vise donc, en tant que produit industriel nouveau, un dérivé du 5-hydroxytryptophane caractérisé en ce qu'il est choisi parmi l'ensemble constitué par

a) le 5-acétyloxy-L-tryptophane, et
b) ses sels d'addition.

L'invention vise également un procédé de préparation du 5-acétyloxy-L-tryptophane à partir du 5-hydroxy-L-tryptophane qui est caractérisé en ce qu'on fait réagir un équivalent de 5-hydroxy-L-tryptophane avec un équivalent d'acide perchlorique dissous dans de l'acide acétique anhydre, on traite un équivalent du produit salifié ainsi obtenu par 1 à 2 équivalents (de préférence 2 équivalents) d'anhydride acétique pour obtenir l'acétate de 5-acétyloxy-L-tryptophane, la neutralisation de ce sel d'addition d'acide, notamment au moyen de $NH_4OH$, donnant le 5-acétyloxy-L-tryptophane. Les autres sels d'addition peuvent être obtenus selon une méthode connue en soi à partir de l'acétate de 5-acétyloxy-L-tryptophane, et de préférence à partir du 5-acétyloxy-L-tryptophane en faisant réagir ce produit avec un acide minéral (notamment HCl, HBr, $H_2SO_4$, $HNO_3$) ou organique (notamment HCOOH, $CH_3CH_2COOH$, $C_6H_5COOH$, ou encore les acides oxalique, fumarique, maléique, malique, ascorbique, p-toluènesulfonique, méthanesulfonique et tartique). Sans sortir du cadre de l'invention on peut également préparer des sels d'addition du type ammonium quaternaire par réaction du 5-acétyloxy-L-tryptophane avec notamment $ICH_3$ ou $ClCH_3$.

Le procédé selon l'invention peut être également mis en oeuvre à partir du 5-hydroxy-DL-tryptophane. On obtient ainsi l'acétate de 5-acétyloxy-DL-tryptophane et le 5-acétyloxy-DL-tryptophane, les isomères L de ces produits étant ensuite, si nécessaire, isolés selon une méthode connue en soi.

D'autres avantages et caractéristiques de l'invention seront mieux compris à la lecture qui va suivre d'exemples de préparation nullement limitatifs, mais donnés à titre d'illustration.

Exemple 1

Acétate de 5-acétyloxy-L-tryptophane monohydrate

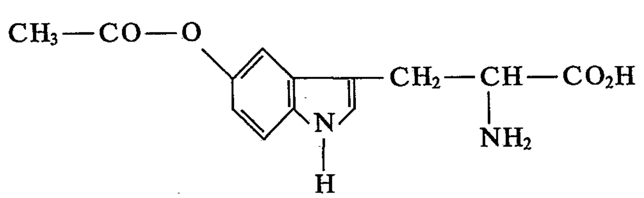

$CH_3COOH$ $H_2O$

— Dans un ballon de 2 litres, dissoudre 25 g de 5-hydroxy-L-tryptophane (0,114 mole) dans 1140 ml d'une solution 0,1 N d'acide perchlorique dans l'acide acétique glacial (totalement exempt d'eau).
— Additionner, goutte à goutte, 114 ml d'une solution à 20% (p/v) d'anhydride acétique (0,22 mole) dans l'acide acétique.

- Porter à 50° C et maintenir à cette température durant 15 minutes.
- Additionner 23 ml d'eau et laisser réagir à 50° C durant 5 minutes.
- Refroidir à température ambiante (15—20° C).
- Ajouter 45 ml d'une solution aqueuse à 40% (p/v) de cyclohexylamine.
- Couler goutte à goutte 1,14 l d'éther diisopropylique dans la solution acétique (dans laquelle le produit à déjà commencé à cristalliser).
- Agiter pendant 1 h 30 après la fin de l'addition.
- Filtrer, rincer avec 180 ml d'un mélange acide acétique-éther diisopropylique (50 : 50) v/v, puis avec 50 ml d'éther diisopropylique.
- Sécher sous vide phosphorique.

On obtient 32,3 g (rendement: 83,7%) d'acétate de 5-acétyloxy-L-tryptophane monohydrate brut.

La purification est effectuée comme indiqué ci-après:

- Dans un erlenmeyer de 200 ml, mettre en suspension 32,3 g du produit brut dans 120 ml du mélange éthanol-eau (50 : 50) v/v.
- Porter 5 minutes au reflux.
- Refroidir à température ambiante (15—20° C).
- Agiter durant 2 heures à 20° C.
- Abandonner une nuit à 5° C.
- Filtrer, rincer avec 2 x 30 ml de mélange eau-éthanol (50 : 50)v/v.
- Sécher sous vide phosphorique.

On obtient 28,2 g (rendement: 87,3%) d'acétate de 5-acétyloxy-L-tryptophane monohydrate purifié. Le point de fusion (déterminé selon la méthode dite au tube capillaire) se situe au voisinage de 225° C.

La présence d'une molécule d'eau dans le produit final purifié est vérifiée par la méthode titrimétrique de K. FISCHER.

Le sel monohydraté se présente sous la forme d'une poudre blanche ou peu colorée, inodore ou d'odeur faible, non hygroscopique vis-à-vis d'une atmosphère de 50° hygrométriques.

Il est soluble dans l'eau: une solution à 1% p/v s'obtient à température ambiante, mais une solution à 2% n'est réalisable qu'à chaud (au bain-marie à 60° C), cette dernière solution restant toutefois totale après retour à température ordinaire.

Dans l'éthanol absolu, ce sel est pratiquement insoluble, même après ébullition du solvant.

Dans le mélange eau-éthanol (50 : 50) v/v, une solution à 1% p/v n'est par réalisable de façon complète en opérant à température ambiante, mais le devient par un chauffage au bain-marie à 60° C et reste complète aprés retour à température ordinaire.

Le spectre d'absorption IR (cf figure 1 ci-après) présente les bandes caractéristiques suivantes: 1750 $cm^{-1}$ (carbonyle ester), 1680 $cm^{-1}$ (acide carboxylique) et 1635 $cm^{-1}$ (carboxylate).

Le pouvoir rotatoire du produit de l'exemple 1 en solution à 2% dans l'acide HCl 2 N est $\alpha_D^{20} = +8°$.

Exemple 2

5-acétyloxy-L-tryptophane

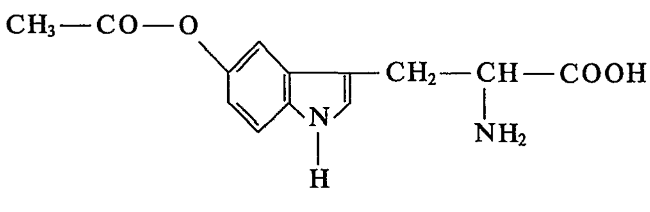

- Dans un bécher de 1 litre, mettre 28,19 g d'acétate de 5-acétyloxy-L-tryptophane monohydrate (purifié comme indiqué à l'exemple 1) en suspension dans 750 ml d'eau.
- Ajouter 6,7 ml d'une solution aqueuse d'ammoniaque à 20,9% (densité: 0,92). On observe une dissolution suivie de la formation d'un épais précipité.
- Agiter pendant 3 heures à 20° C.
- Filtrer puis rincer avec 2 x 30 ml d'eau.

On obtient ainsi 15,2 g (rendement: 70%) de 5-acétyloxy-L-tryptophane. Le point de fusion (déterminé selon la méthode dite au tube capillaire) se situe au voisinage de 220° C.

Ce produit se présente sous la forme d'une poudre blanche ou peu colorée, inodore ou d'odeur faible, non hygroscopique vis-à-vis d'une atmosphère de 70° hygrométriques.

Il est soluble dans l'eau, toutefois à condition d'opérer à chaud pour des concentrations aussi bien

de 1% que de 2% p/v (au bain-marie à 60° C), mais ces deux solutions restent totales après retour à température ordinaire.

Dans l'éthanol anhydre, cet ester est pratiquement insoluble même après ébullition du solvant.

Dans le mélange eau-éthanol (50 : 50) v/v, une solution à 2% p/v n'est pas réalisable de façon complète en opérant à température ambiante, mais le devient par un chauffage au bain-marie à 60° C et reste complète après retour à température ordinaire.

Le spectre d'absorption IR (cf. figure 2 ci-après) présente les bandes caractéristiques suivantes: 1735 $cm^{-1}$ (carbonyle ester) et 1605 $cm^{-1}$ (carboxylate).

Le pouvoir rotatoire du produit de l'exemple 2 en solution dans de l'acide HCl 2 N est $\alpha_D^{20} = +9°$.

On a résumé ci-après les résultats des essais pharmacologiques qui ont été entrepris.

## TOXICITE

### 1) Etude par voie intrapéritonéale chez 10 souris mâles

— 5-acétyloxy-L-tryptophane à la dose de 800 mg/kg.
1 h après administration: 2 morts/10
24 h après administration: 2 morts/8.

Au total, sur 10 jours: 4 morts/10

— Produit de l'exemple 1 à la dose de 800 mg/kg chez 10 souris mâles.
1 h après administration: 0 mort
24 h après administration: 1 mort
jours suivants: 0 mort.

Au total, 1 mort sur 10.

### 2) Etude par voie orale chez 10 souris mâles

— 5-acétyloxy-L-tryptophane à la dose de 1 g/kg.
1 h après administration: 2 morts
24 h après administration: 0 mort
10 jours après administration: 0 mort.

Au total, 2 morts/10 dans l'heure qui suit l'administration.

— Produit de l'exemple 1 à la dose de 1 g/kg.

Au total, aucun mort dans les heures et les 10 jours suivants.

## PHARMACOLOGIE

La mise en évidence chez l'animal de manifestations motrices caractéristiques après administration de L-5-hydroxytryptophane à dose suffisante, a déjà fait l'objet de nombreux travaux. Ces manifestations sont attribuées à une activation des récepteurs sérotoninergiques cérébraux chez des animaux prétraités aux IMAO.

Dans les conditions expérimentales adoptées, le 5-acétyloxy-L-tryptophane entraîne, ainsi que le produit de l'exemple 1, des modifications du comportement moteur, chez la souris, plus importantes que celles que provoque une dose équimoléculaire de 5-hydroxy-L-tryptophane.

Les études pharmacologiques, réalisées, conduisent à penser que la concentration cérébrale en sérotonine est plus rapide et plus importante après administration de 5-acétyloxy-L-tryptophane et de ses sels, qu'après administration de 5-hydroxy-L-tryptophane.

Les applications cliniques, qui découlent des constatations pharmacologiques, concernent les déficits en sérotonine responsables de désordres neuropsychiatriques tels que:

— dysthymies, en particulier de syndromes dépressifs (avec association possible d'antidépresseurs),
— troubles du sommeil (effet inducteur),
— certains mouvements anormaux.

La posologie moyenne préconisée chez l'homme est de l'ordre de 1,5 à 2 g par voie orale et de 0,5 g par voie parentérale, par jour, de 5-acétyloxy-L-tryptophane ou d'un de ses sels d'addition.

**Revendications**

1. Nouveau dérivé de 5-hydroxy-L-tryptophane caractérisé en ce qu'il est choisi parmi l'ensemble constitué par:

a) le 5-acétyloxy-L-tryptophane, et
b) ses sels d'addition.

2. 5-acétyloxy-L-tryptophane.
3. Acétate de 5-acétyloxy-L-tryptophane monohydrate.
4. Procédé de préparation du 5-acétyloxy-L-tryptophane à partir du 5-hydroxy-L-tryptophane, caractérisé en ce qu'on fait réagir un équivalent de 5-hydroxy-L-tryptophane avec un équivalent d'acide perchlorique dissous dans de l'acide acétique anhydre, on traite un équivalent, du produit salifié ainsi obtenu par 1 à 2 équivalents (de préférence 2 équivalents) d'anhydride acétique pour obtenir l'acétate de 5-acétyloxy-L-tryptophane, la neutralisation de ce sel d'addition d'acide, notamment au moyen de $NH_4OH$, donnant le 5-acétyloxy-L-tryptophane.
5. Composition thérapeutique caractérisée en ce qu'elle renferme en association avec un excipient physiologiquement acceptable, au moins un composé choisi parmi l'ensemble constitué par le 5-acétyloxy-L-tryptophane et ses sels d'addition, en tant qu'ingrédient actif.

**Patentansprüche**

1. Neues 5-Hydroxy-L-tryptophan-Derivat, dadurch gekennzeichnet, daß es ausgewählt wird aus der Gruppe, die besteht aus:

a) dem 5-Acetyloxy-L-tryptophan und
b) seinen Additionssalzen.

2. 5-Acetyloxy-L-tryptophan.
3. Acetat von 5-Acetyloxy-L-tryptophanmonohydrat.
4. Verfahren zur Herstellung von 5-Acetyloxy-L-tryptophan aus 5-Hydroxy-L-tryptophan, dadurch gekennzeichnet, daß man ein Äquivalent 5-Hydroxy-L-tryptophan mit einem Äquivalent Perchlorsäure, gelöst in wasserfreier Essigsäure, reagieren läßt, ein Äquivalent des dabei erhaltenen Salzprodukts mit 1 bis 2 Äquivalenten (vorzugsweise 2 Äquivalenten) Essigsäureanhydrid behandelt unter Bildung von 5-Acetyloxy-L-tryptophanacetat und dieses Säureadditionssalz neutralisiert, insbesondere mit $NH_4OH$, unter Bildung von 5-Acetyloxy-L-tryptophan.
5. Therapeutische Zusammensetzung, dadurch gekennzeichnet, daß sie zusammen mit einem physiologisch akzeptablen Exzipient als Wirkstoff mindestens eine Verbindung enthält, die ausgewählt wird aus der Gruppe, die besteht aus 5-Acetyloxy-L-tryptophan und seinen Additionssalzen.

**Claims**

1. A novel 5-hydroxy-L-tryptophane derivative selected from the group consisting of:

a) 5-acetyloxy-L-tryptophane, and
b) its addition salts.

2. 5-acetyloxy-L-tryptophane.
3. Acetate of 5-acetyloxy-L-tryptophane monohydrate.
4. A method for the preparation of 5-acetyloxy-L-tryptophane from 5-hydroxy-L-tryptophane, which comprises reacting an equivalent of 5-hydroxy-L-tryptophane with an equivalent of perchloric acid in solution in anhydrous acetic acid, in treating an equivalent of the resulting salified product with 1 or 2 equivalents (2 preferably) of acetic anhydride to obtain 5-acetyloxy-L-tryptophane acetate, the neutralization of this acid addition salt, in particular with $NH_4OH$ giving 5-acetyloxy-L-tryptophane.
5. A therapeutical composition useful in neuropsychiatry for treating serotonin deficiencies, containing, in association with a physiologically acceptable excipient, a pharmaceutically effective amount of a compound selected from the group consisting of 5-acetyloxy-L-tryptophane and its addition salts, as active ingredient.

Fig-1

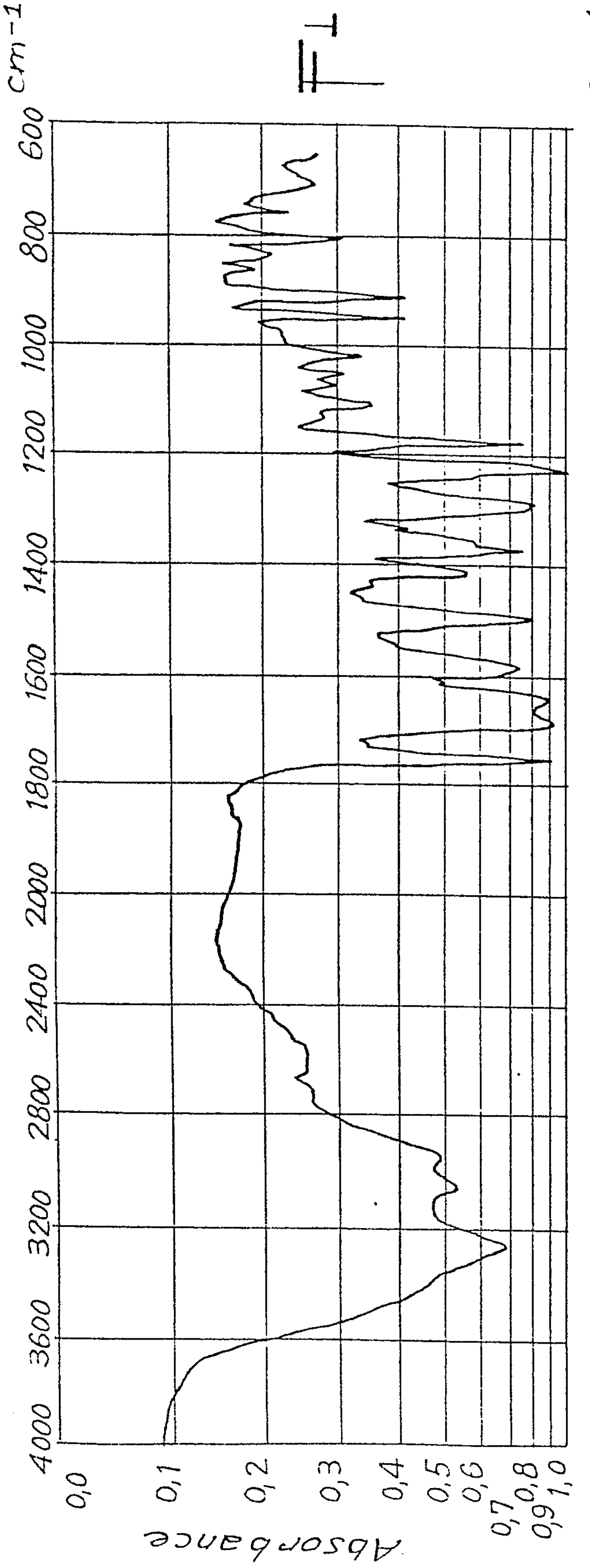
cm-1
600
800
1000
1200
1400
1600
1800
2000
2400
2800
3200
3600
4000
0,0
0,1
0,2
0,3
0,4
0,5
0,6
0,7
0,8
0,9
1,0
Absorbance

Fig-2

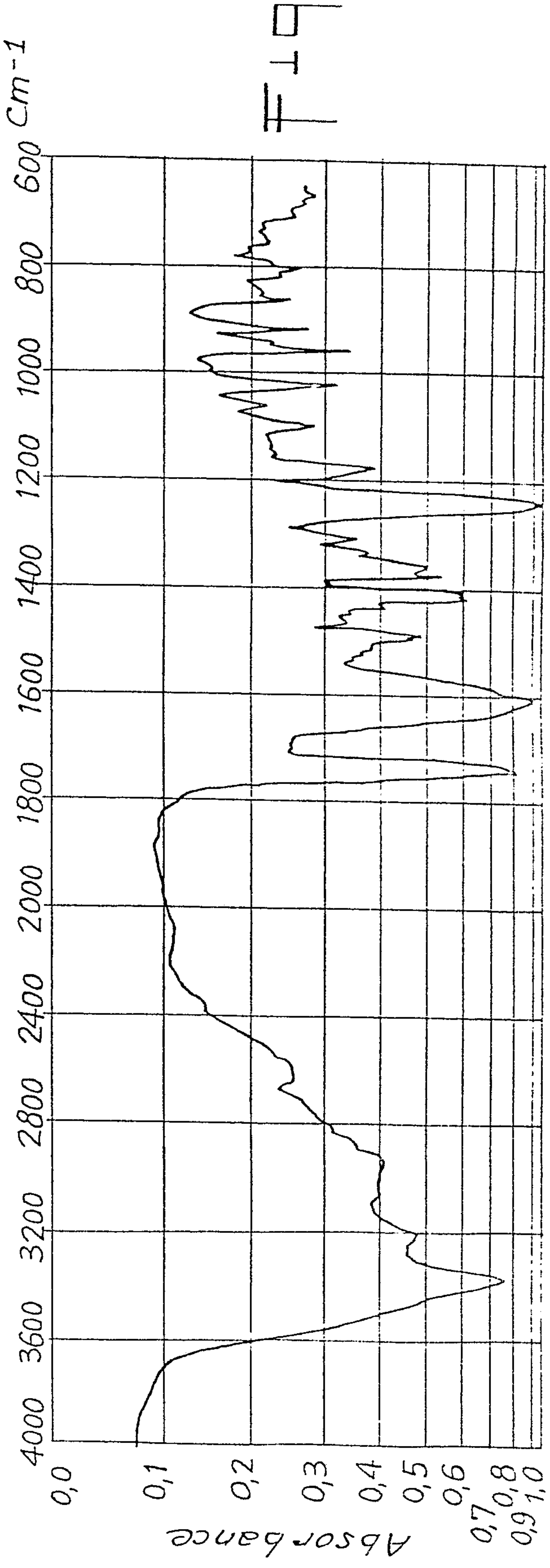
Cm-1
600
800
1000
1200
1400
1600
1800
2000
2400
2800
3200
3600
4000
0,0
0,1
0,2
0,3
0,4
0,5
0,6
0,7
0,8
0,9
1,0
Absorbance